(19) Europäisches Patentamt
European Patent Office
Office européen des brevets

(11) Veröffentlichungsnummer: **0 296 381**
**A1**

(12) # EUROPÄISCHE PATENTANMELDUNG

(21) Anmeldenummer: 88108603.7

(51) Int. Cl.[4]: C07D 231/44

(22) Anmeldetag: 30.05.88

(30) Priorität: 12.06.87 DE 3719732

(43) Veröffentlichungstag der Anmeldung: 28.12.88 Patentblatt 88/52

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Jensen-Korte, Uta, Dr.
Geibelstrasse 9
D-4000 Düsseldorf 1(DE)
Erfinder: Schallner, Otto, Dr.
Noldeweg 22
D-4019 Monheim(DE)
Erfinder: Stetter, Jörg, Dr.
Gellertweg 4
D-5600 Wuppertal 1(DE)
Erfinder: Becker, Benedikt, Dr.
Metzkausener Strasse 14
D-4020 Mettmann(DE)
Erfinder: Behrenz, Wolfgang, Dr.
Untergründemich 14
D-5063 Overath(DE)
Erfinder: Stendel, Wilhelm, Dr.
In den Birken 55
D-5600 Wuppertal 1(DE)
Erfinder: Andrews, Peter, Dr.
Gellertweg 2
D-5600 Wuppertal 1(DE)

(54) **Substituierte 5-Methylamino-1-arylpyrazole.**

(57) Es wurden neue substituierte 5-Methylamino-1-arylpyrazole der allgemeinen Formel (I)

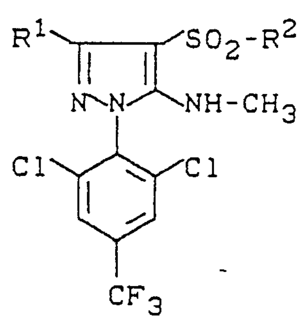

(I)

in welcher
R' für Alkyl steht und

EP 0 296 381 A1

$R^2$ für Halogenalkyl steht,

bereitgestellt.

Die neuen Verbindungen der Formel (I) besitzen eine stark ausgeprägte Wirksamkeit gegenüber tierischen Schädlingen, insbesondere gegen Insekten und Nematoden. Die neuen Verbindungen sind daher sehr gut als Wirkstoffe in Schädlingsbekämpfungsmitteln geeignet.

## Substituierte 5-Methylamino-1-arylpyrazole

Die Erfindung betrifft neue substituierte 5-Methylamino-1-arylpyrazole, mehere Verfahren zu ihrer Herstellung und ihre Verwendung als Schädlingsbekämpfungsmittel.

Es ist bereits bekannt, daß bestimmte 1-Arylpyrazole wie beispielsweise das 1-(2,6-Dichlor-4-triluormethylphenyl)-5-methylamino-4-trifluormethylthio-pyrazol oder das 1-(2,6-Dichlor-4-trifluormethylphenyl)-5-methylamino-4-dichlorfluormethylthio-pyrazol insektizide, akarizide und nematizide Eigenschaften besitzen (vgl. EP 201 852).

Die Wirksamkeit dieser vorbekannten Verbindungen ist jedoch insbesondere bei niedrigen Aufwandmengen und Konzentrationen nicht in allen Anwendunsbereichen völlig zufriedenstellend.

Es wurden neue 1-Arylpyrazole der allgemeinen Formel (I),

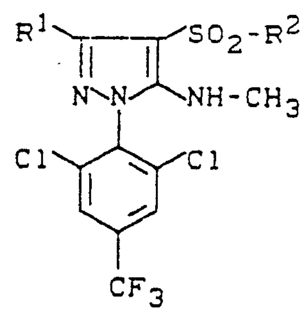

(I)

in welcher
R' für Alkyl steht und
$R^2$ für Halogenalkyl steht,
gefunden.

Weiterhin wurde gefunden, daß man die neuen 1-Arylpyrazole der allgemeinen Formel (I),

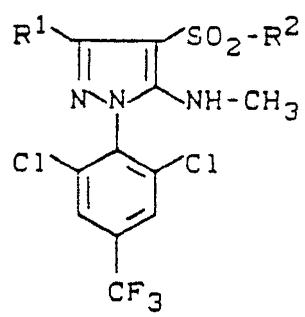

(I)

in welcher
R' für Alkyl steht und
$R^2$ für Halogenalkyl steht,
erhält, wenn man

(a) 5-Amino-1-aryl-pyrazole der Formel (II),

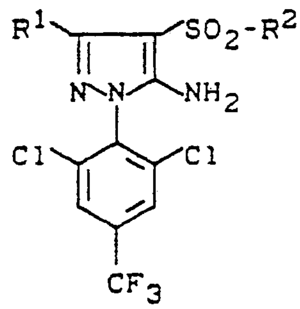

(II)

in welcher
$R^1$ und $R^2$ die oben angegebene Bedeutung haben,
mit Methylierungsmitteln der Formel (III),

$CH_3$-E (III)

in welcher
E für eine elektronenanziehende Abgangsgruppe steht,
gegebenenfalls in Gegenwart eines Verdünnungsmittels und gegebenenfalls in Gegenwart eines Säurebindemit tels sowie gegebenenfalls in Gegenwart eines Katalysators umsetzt : oder wenn man

(b) 5-(N-Acylamino)-1-arylpyrazole der Formel (IV),

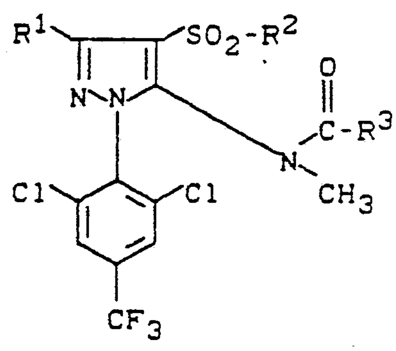

(IV)

in welcher
$R^1$ und $R^2$ die oben angegebene Bedeutung haben und
$R^3$ für Alkyl steht,
mit Säuren als Katalysator gegebenenfalls in Gegenwart eines Verdünnungsmittels deacyliert; oder wenn man

(c) 1-Arylpyrazole der Formel (V),

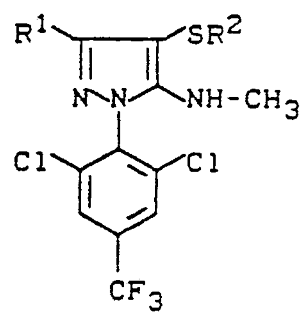

(V)

in welcher
$R^1$ und $R^2$ die oben angegebene Bedeutung haben,
mit einem Oxidationsmittel, gegebenenfalls in Gegenwart eines Verdünnungsmittels und gegebenenfalls in Gegenwart eines Katalysators sowie gegebenenfalls in Gegenwart eines Säurebindemittels umsetzt; oder wenn man

(d) 5-Halogen-1-aryl-pyrazole der Formel (VI),

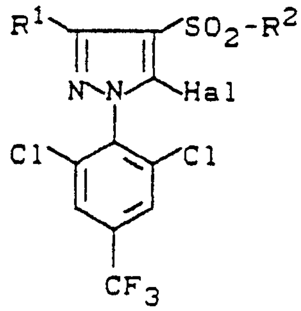

(VI)

in welcher
$R^1$ und $R^2$ die oben angegebene Bedeutung haben und
Hal für Halogen steht,
mit Methylamin der Formel (VII),

$CH_3$-$NH_2$ (VII)

gegebenenfalls in Gegenwart eines Verdünnungsmittels und gegebenenfalls in Gegenwart eines Säurebindemittels umsetzt.

Schließlich wurde gefunden, daß die neuen substituierten 5-Methylamino-1-arylpyrazole der Formel (I) eine sehr gute Wirkung gegen tierische Schädlinge besitzen.

Überraschenderweise zeigen die erfindungsgemäßen substituierten 5-Methylamino-1-arylpyrazole eine erheblich bessere Wirkung gegen tierische Schädlinge als die aus dem Stand der Technik bekannten 1-Arylpyrazole, wie beispielsweise das 1-(2,6-Dichlor-4-trifluormethylphenyl)-5-methylamino-4-dichlorfluormethylthio-pyrazol oder das 1-(2,6-Dichlor-4-trifluormethylphenyl)-5-methylamino-4-trifluormethylthio-pyrazol, welche chemisch und wirkungsmäßig naheliegende Verbindungen sind.

Die erfindungsgemäßen substituierten 5-Methylamino-1-arylpyrazole sind durch die Formel (I) allgemein definiert. Bevorzugt sind Verbindungen der Formel (I), bei welchen
$R^1$ für geradkettiges oder verzweigtes Alkyl mit 1 bis 4 Kohlenstoffatomen steht und
$R^2$ für geradkdkettiges oder verzweigtes Halogenalkyl mit 1 bis 4 Kohlenstoffatomen und 1 bis 9 gleichen oder verschiedenen Halogenatomen steht.

Besonders bevorzugt sind Verbindungen der Formel (I), bei welchen
$R^1$ für Methyl oder Ethyl steht und
$R^2$ für Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl . Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl oder Fluorchlorbrommethyl steht.

Ganz besonders bevorzugt sind Verbindungen der Formel (I), bei welchen
$R^1$ für Methyl steht und
$R^2$ für Trifluormethyl, Dichlorfluormethyl oder Difluorchlormethyl steht.

Verwendet man beispielsweise 5-Amino-3-methyl-4-trifluormethylsulfonyl-1-(2,6-dichlor-4-trifluormethylphenyl)pyrazol und Methyliodid als Ausgangsstoffe, so läßt sich der Reaktionsablauf des erfindungsgemäßen Verfahrens (a) durch das folgende Formelschema darstellen:

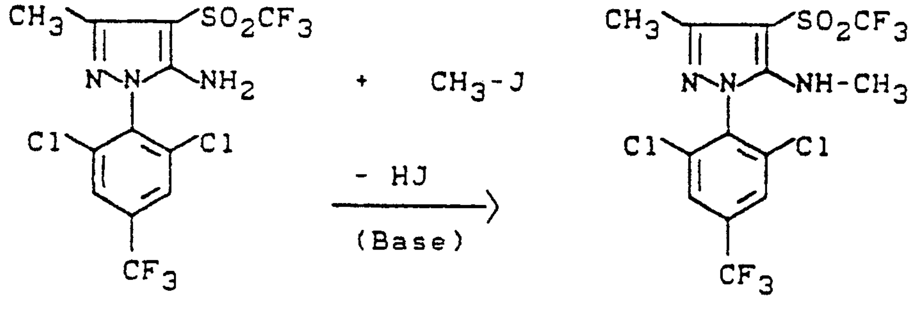

Verwendet man beispielsweise 3-Methyl-4-trifluormethylsulfonyl-5-(N-methyl-acetamido)-1-(2,6-dichlor-4-trifluormethylphenyl)-pyrazol als Ausgangsverbindung, so läßt sich der Reaktionsablauf des Erfindungsgemäßen Verfahrens (b) durch das folgende Formelschema darstellen:

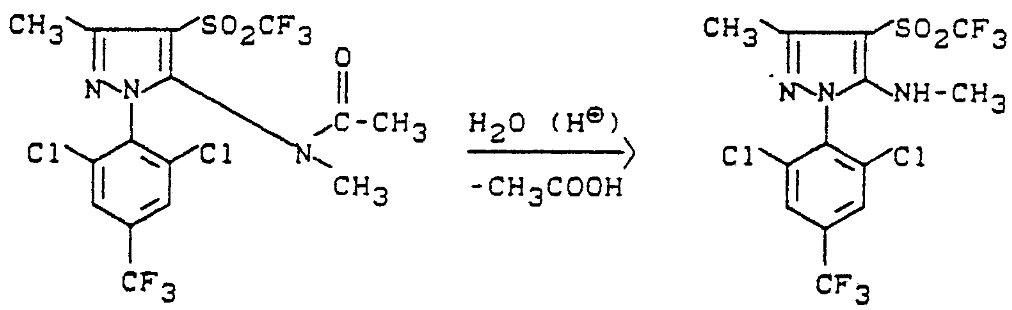


Verwendet man beispielsweise 3-Methyl-5-methylamino-4-trifluormethylthio-1-(2,6-dichlor-4-trifluormethylphenyl)-pyrazol als Ausgangsverbindung und m-Chlorperbenzoesäure als Oxidationsmittel, so läßt sich der Reaktionsablauf des erfindungemäßen Verfahrens (c) durch das folgende Formelschema darstellen:

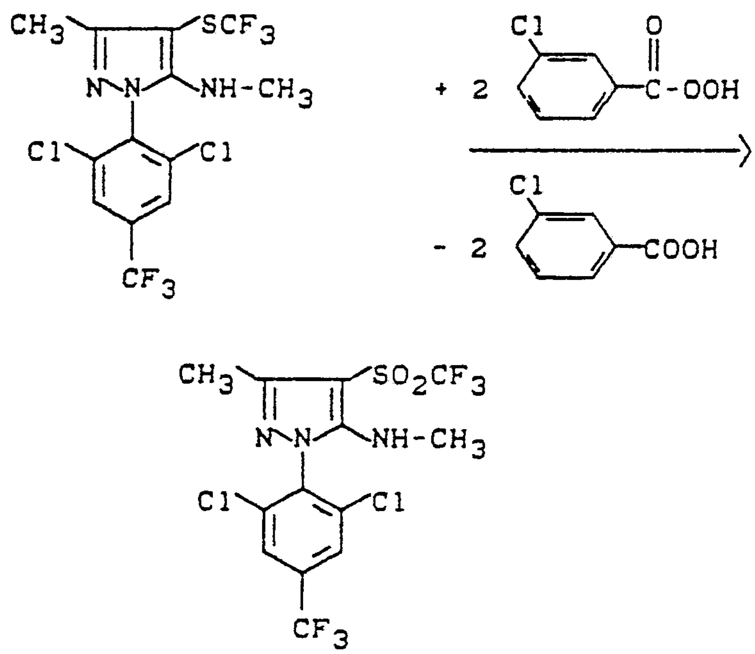


Verwendet man beispielsweise 4-Dichlorfluormethylsulfonyl-5-brom-3-methyl-1-(2,6-dichlor-4-trifluormethylphenyl)-pyrazol und Methylamin als Ausgangsstoffe, so läßt sich der Reaktionsablauf des erfindungsgemäßen Verfahrens (d) durch das folgende Formelschema darstellen:

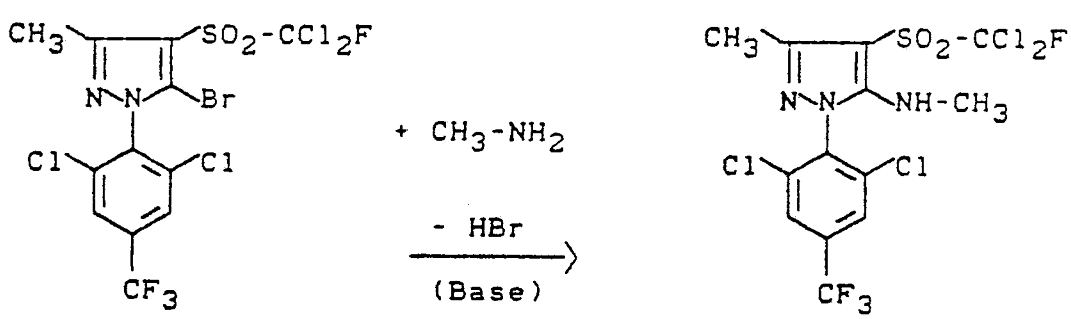

Die zur Durchführung des erfindungsgemäßen Verfahrens (a) als Ausgangsstoffe benötigten 5-Amino-1-arylpyrazole sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind bekannt (vgl. EP 201 852).

Die zur Durchführung des erfindungsgemäßen Verfahrens (a) weiterhin als Ausgangsstoffe benötigten Methylierungsmittel sind durch die Formel (III) allgemein definiert. In dieser Formel (III) steht E vorzugsweise für Halogen, insbesondere für Chlor, Brom oder Iod oder für Methoxysulfonyloxy oder p-Toluolsulfonyloxy.

Die Methylierungsmittel der Formel (III) sind allgemein bekannte Verbindungen der organischen Chemie.

Die zur Durchführung des erfindungsgemäßen Verfahrens (b) als Ausgangsstoffe benötigten 5-(N-acylamino)-1-arylpyrazole sind durch die Formel (IV) allgemein definiert. In dieser Formel (IV) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

$R^3$ steht vorzugsweise für geradkettiges oder verzweigtes Alkyl mit 1 bis 4 Kohlenstoffatomen, insbesondere für Methyl oder Ethyl.

Die 5-(N-Acylamino)-1-aryl-pyrazole der Formel (IV) sind noch nicht bekannt. Man erhält sie jedoch in Analogie zu bekannten Verfahren (vgl. z.B. EP 201 852), wenn man 5-(N-Acylamino)-pyrazole der Formel (VIII),

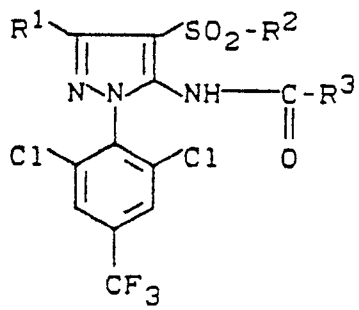

(VIII)

in welcher

$R^1$, $R^2$ und $R^3$ die oben angegebene Bedeutung haben,

mit Methylierungsmitteln der Formel (III),

$CH_3$-E (III)

in welcher

E für eine elektronenanziehende Abgangsgruppe, wie beispielsweise Halogen, insbesondere für Chlor, Brom oder Iod oder für Methoxysulfonyloxy oder p-Toluolsulfonyloxy steht,

in Analogie zur Durchführung des erfindungsgemäßen Verfahrens (a) gegebenenfalls in Gegenwart eines Verdünnungsmittels wie beispielsweise Dichlormethan und gegebenenfalls in Gegenwart einer Base wie beispielsweise Natronlauge sowie gegebenenfalls in Gegenwart eines Phasentransferkatalysators wie beispielsweise Tributylbenzylammoniumchlorid bei Temperaturen zwischen 0 °C und 120 °C umsetzt.

Die 5-(N-Acylamino)-pyrazole der Formel (VIII) sind bekannt (vgl. EP 201 852).

Die zur Durchführung des erfindungsgemäßen Verfahrens (c) als Ausgangsstoffe benötigten 1-Arylpyrazole sind durch die Formel (V) allgemein definiert. In dieser Formel (V) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (V) sind bekannt oder erhältlich in Analogie zu bekannten Verfahren (vgl. EP 201 852), beispielsweise wenn man 5-Amino-1-arylpyrazole der Formel (IX),

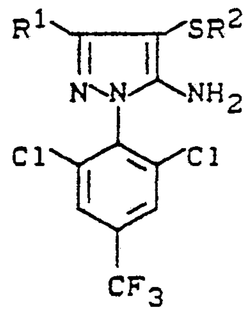

(IX)

in welcher
$R^1$ und $R^2$ die oben angegebene Bedeutung haben,
zunächst mit Acylierungsmitteln der Formel (X),

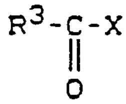

(X)

in welcher
$R^3$ für Alkyl, insbesondere für Methyl oder Ethyl steht und
X für eine elektronenanziehende Abgangsgruppe wie Halogen oder Alkylcarbonyloxy, insbesondere für Chlor, Acetoxy oder Propionyloxy steht,
gegebenenfalls in Gegenwart eines Verdünnungsmittels wie beispielsweise Dichlormethan und gegebenenfalls in Gegenwart eines Säurebindemittels wie beispielsweise Triethylamin bei Temperaturen zwischen - 20 °C und + 120 °C umsetzt, dann die so erhältlichen 5-(N-Acrylamino)-1-arylpyrazole der Formel (XI),

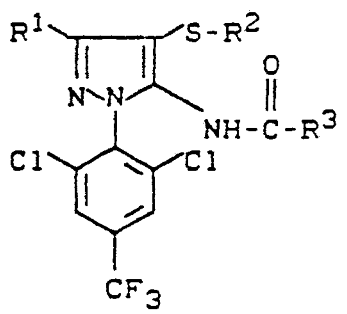

(XI)

in welcher
$R^1$, $R^2$ und $R^3$ die oben angegebene Bedeutung haben,
mit Methylierungsmitteln der Formel (III),
$CH_3$-E (III)
in welcher
E für eine elektronenanziehende Abgangsgruppe, wie, insbesondere für lod, Methoxysulfonyloxy oder p-Toluolsulfonyloxy steht,
in Analogie zur Durchführung des erfindungsgemäßen Verfahrens (a) gegebenenfalls in Gegenwart eines Verdünnungsmittels wie beispielsweise Acetonitril und gegebenenfalls in Gegenwart eines Säurebindemittels wie beispielsweise Kaliumcarbonat be Temperaturen zwischen 0 °C und 180 °C methyliert und - schließlich die so erhältichen 1-Arylpyrazole der Formel (XII),

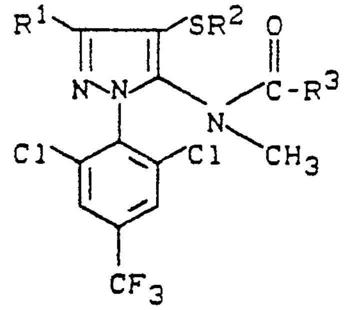

(XII)

in welcher

$R^1$, $R^2$ und $R^3$ die oben angegebene Bedeutung haben, in Analogie zur Durchführung des erfindungsgemäßen Verfahrens (b) mit Säuren wie beispielsweise Schwefelsäure gegebenenfalls in Gegenwart eines Verdünnungsmittels wie beispielsweise Methanol bei Temperaturen zwischen 0 °C und 120 °C deacyliert.

Die 5-Amino-1-aryl-pyrazole der Formel (IX) sind bekannt ebenso wie die 5-(N-Acrylamino)-1-arylpyrazole der Formel (XI) (vgl. EP 201 852).

Die Acylierungsmittel der Formel (X) sind allgemein bekannte Verbindungen der organischen Chemie.

Die zur Durchführung des erfindungsgemäßen Verfahrens (d) als Ausgangsstofe benötigten 5-Halogen-1-arylpyrazole sind durch die Formel (VI) allgemein definiert. In dieser Formel (VI) stehen R' und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Hal steht vorzugsweise für Chlor oder Brom.

Die 5-Halogen-1-aryl-pyrazole der Formel (VII) sind bekannt (vgl. DE-OS 3 529 829).

Das zur Durchführung des erfindungsgemäßen Verfahrens (d) weiterhin als Ausgangsverbindung benötigte Methyl amin der Formel (VII) ist eine allgemein bekannte Verbindung der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder d-eithylether, Ketone wie Acetone oder Butanon, Nitrile, wie Acetonitril oder Propionitril, Amide, wie Dimethylformamid, Dimethylacetamid, N-Methylformanilid, N-Methylpyrrolidon oder Hexamethylphophorsäuretriamid, Ester, wie Essigsäureethylester oder Sulfoxide, wie Dimethylsulfoxid.

Das erfindunsgemäße Verfahren (a) kann gegebenenfalls auch in einem Zweiphasensystem, wie beispielsweise Wasser Toluol oder Wasser Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des erfindungsgemäßen Verfahrens (a) kommen alle üblicherweise verwendbaren anorganischen und organischen Basen infrage, Vorzugsweise verwendet man Alkalimetallhydride, -hydroxide, -amide, alkholate, -carbonate oder hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)pyridin, Diazabicyclooctan (DABCO), Diazabicyclononen (DBN) oder Diazabicycloundecan (DBU).

Die Reaktionstemperaturen können bei der Durchführung des erfindungsgemäßen Verfahrens (a) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (a) setzt man pro Mol 5-Amino-1-aryl-pyrazol der Formel (II) im allgemeinen 1.0 bis 20.0 Mol, vorzugsweise 1.0 bis 15.0 Mol an Methylierungsmittel der Formel (III) und gegebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen anorganische oder organische polare Lösungsmittel in Frage. Vorzugsweise verwendet man Alkohole, wie beispielsweise

Methanol, Ethanol oder Propanol, oder deren Gemische mit Wasser oder reines Wasser als Verdünnungsmittel.

Als Katalysatoren zur Durchführung des erfindungsgemäßen Verfahrens (b) kommen vorzugsweise anorganische Mineralsäuren, insbesondere Chlorwasserstoffsäure oder schwefelsäure infrage.

Die Reaktionstemperaturen können bei der Durchführung des erfindungsgemäßen Verfahrens (b) in einem größeren Bereich variiert werden. Im allgemeinen arbeitet man zwischen +20 °C und +150 °C, vorzugsweise zwischen +50 °c und +120 °C.

Zur Durchführung des erfindungsgemäßen Verfahrens (b) setzt man pro Mol 5-(N-Acylamino)-1-arylpyrazol der Formel (IV) im allgemeinen 1,0 bis 20,0 Mol, vorzugsweise 1,0 bis 10,0 Mol an Katalysatorsäure ein und erwärmt für mehrere Stunden auf die erforderliche Reaktionstemperatur. Die Aufarbeitung, Isolierung und Reinigung der Reaktionsprodukte der Formel (I) erfolgt nach üblichen Methoden.

Als Oxidationsmittel zur Durchführung des erfindungsgemäßen Verfahrens (c) kann man alle üblicherweise für Schwefeloxidationen infrage kommenden anorganischen oder organischen Oxidationsmittel verwenden. Vorzugsweise verwendet man organische Persäuren, wie beispielsweise Peressigsäure, 4-Nitroperbenzoesäure oder 3-Chlorperbenzoesäure, anorganische Persäuren, wie beispielsweise Periodsäure oder auch Wasserstoffperoxid, Kaliumpermanganat oder Chromsäure.

Als Verdünnungsmittel zur Durchführung des erfindungsgemäßen Verfahrens (c) kommen ebenfalls inerte organische Lösungsmittel infrage.

Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (c) kann gegebenenfalls in Gegenwart eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxid, -acetate oder -carbonate, wie beispielsweise Calciumhydroxid, Natriumhydroxid, Natriumacetat oder Natriumcarbonat.

Das erfindungsgemäße Verfahren (c) kann gegebenenfalls in gegenwart eines geeigneten Katalysators durchgeführt werden. Als solche kommen alle üblicherweise für derartige Schwefeloxidationen üblichen Katalysatoren infrage. Beispielhaft genannt seien in diesem Zusammenhang Schwermetallkatalysatoren wie Ammoniummolybdat.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +70 °C, vorzugsweise bei Temperaturen zwischen 0 °C und +50 °C.

Zur Durchführung des erfindungsgemäßen Verfahrens (c) setzt man pro Mol an 1-Aryl-pyrazol der Formel (V) im allgemeinen 1.8 bis 5.0 Mol, vorzugsweise doppelt molare Mengen an Oxidationsmittel ein. Die Reaktionsführung, Aufarbeitung und Isolierung der Endprodukte der Formel (I) erfolgt nach üblichen Verfahren.

Als Verdünnungsmittel zur Durchführung des erfindungsgemäßen Verfahrens (d) kommen inerte organische Lösungsmittel infrage. Hierzu gehören insbesondere aliphatische, alicyclislche oder aromatische, gegebenenfalls halogenierte Kohlenwasserstofe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide wie Dimethylsulfoxid.

Das erfindungsgemäße Verfahren (d) kann gegebenenfalls in Gegenwart eines geeigneten Säurebindemittels durchgeführt werden. Als solche kommen alle üblichen anorganischen oder organischen Basen infrage. Hierzu gehören beispielsweis Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Methylamin der Formel (VII) gleichzeitig als Säurebindemittel zu verwenden.

Die Reaktionstemperaturen können bei der Durchführung des erfindungsgemäßen Verfahrens (d) in einem größeren Bereich variiert werden. Im allgemeinen arbeitet man bei Temperaturen zwischen -20 °C und +200 °C, vorzugsweise bei Temperaturen zwischen 0 °C und +150 °C.

Zur Durchführung des erfindungsgemäßen Verfahrens (d) setzt man pro Mol an 5-Halogen-1-aryl-pyrazol der Formel (VI) im allgemeinen 1.0 bis 10.0 Mol, vorzugsweise 1.0 bis 5.0 Mol an Methylamin der Formel (VII) ein. Die Reaktionsdurchführung, Aufarbeitung und Isolierung der Reaktionsprodukte der Formel (I) erfolgt nach allgemein üblichen Verfahren.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella Germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura zB. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Tirchopulsia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homonoa magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp.. Tenebrio molitor Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratities capitate, Dacus oleae, Tripula paludosa.

Aus der Ordnung der Siphonaptera z.B. Xenopsylla cheopis, Ceratophyllus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe wirken nicht nur gegen Pflanzen-, Hygiene- und Voratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten und Endoparasiten wirksam.

Die erfindungsgemäß verwendbaren Wirkstoffe der Formel (I) zeichnen sich durch eine hohe insektizide Wirksamkeit aus. Sie lassen sich insbesondere gegen pflanzenschädigende Insekten, wie beispielsweise gegen die Larven der Meeretttichblattkäfer (Phaedon cochleariae) einsetzen. Sei eignen sich daneben auch hervorragend zur Bekämpfung von Bodeninsekten.

Außerdem besitzen die erfindungsgemäß verwendbaren Wirkstoffe der Formel (I) eine hohe Wirkung gegen Hygiene- und Vorratsschädlinge und lassen sich beispielsweise zur Bekämpfung von Mückenlarven (Aedes aegypti) einsetzen.

Darüber hinaus lassen sich die erfindungsgemäßen Wirkstoffe mit besonders gutem Erfolg zur Bekämpfung von parasitisch lebenden Warmblüterschädlingen, wie beispielsweise gegen die Larven der Goldfliege (Lucilia cuprina), gegen Stechfliegen (Stomoxys calcitrans), gegen die Weideviehfliege (Musca autumnalis) oder gegen Rinderzecken (Boophilus microplus) sowie gegen endoparasitisch lebende Nematoden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermit teln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ether, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstofe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und oder - schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalter im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilan tien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den

aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen ,0001 und 1 Gew.-% liegen. Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höher Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgeießens (pour-on and spot-on) und des Einpuderns sowie durch parenterale Anwendung in Form beispielsweise der Injektion sowie ferner durch das "feedthrough"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden:

## Herstellungsbeispiele

### Beispiel 1

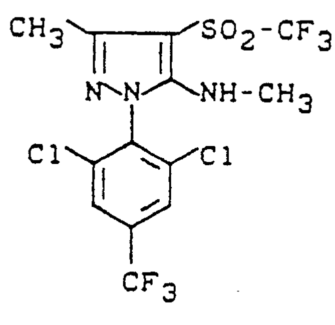

(Verfahren b)

5,1 g (0,01 Mol) 5-(N-Propionyl-N-methyl-amino)-3-methyl-4-trifluormethylsulfonyl-1-(2,6-dichlor-4-trifluormethylphenyl)-pyrazol werden in 30 ml 80prozentiger wässriger Schwefelsäure 20 Stunden bei Raumtemperatur gerührt. Zur Aufarbeitung gießt man die Reaktionsmischung auf Eiswasser, extrahiert mehrfach mit Dichlormethan, wäscht die vereinigten organischen Phasen mit gesättigter wässriger Natriumhydrogencarbonatlösung, trocknet über Magnesiumsulfat und entfernt das Lösungsmittel im Vakuum.

Man erhält 4 g (88 % der Theorie) an 5-Methylamino-3-methyl-4-trifluormethylsulfonyl-1-(2,6-dichlor-4-trifluormethyl)pyrazol vom Schmelzpunkt 139 °C - 140 °C.

alternative Herstellung:

(Verfahren c)

Zu 25 g (0,059 Mol) 5-Methylamin-3-methyl-4-trifluormethylthio-1-(2,6-dichlor-4-trifluormethylphenyl)-pyrazol (vgl. EP 201 852) in 80 ml 80 prozentiger Schwefelsäure gibt man bei Raumtemperatur 20,6 (0,18 Mol) 30rozentige wässrige Wasserstoffperoxidlösung, rührt 12 Stunden bei 60 °C, gießt die Reaktionsmischung im Eiswasser, extrahiert mit Dichlormethan, wäscht mit wässriger Natriumhydrogencarbonatlosung, trocknet über Magnesiumsulfat und entfernt das Lösungsmittel im Vakuum.

Man erhält 20 g (74 % der Theorie) an 5-Methylamino-3-methyl-4-trifluormethylsulfonyl-1-(2,6-dichlor-4-trifluormethylphenyl)-pyrazol vom Schmelzpunkt 139 °C -140 °C.

Herstellung der Ausgangsverbindung

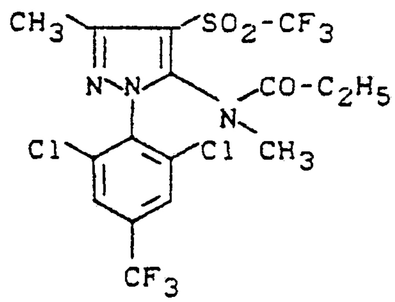

Zu 149,4 g (0,3 Mol) 3-Methyl-5-propionamido-1-(2,6-dichlor-4-trifluormethylphenyl)-4-trifluormethylsulfonyl-pyrazol (vgl. EP 201 852) in 950 ml Dichlormethan gibt man unter Rühren bei Raumtemperatur 237 ml 45prozentige wässrige Natriumhydroxidlösung, 0,5 g Tributylbenzylammoniumchlorid und 30 ml (0,315 Mol) Dimethylsulfat, rührt 2 Stunden bei Raumtemperatur, gibt 500 ml Dichlormethan und 300 ml Wasser zu und rührt weitere 30 minuten. Zur Aufarbeitung wird die organische Phase abgetrennt, mit 500 ml 2prozentiger Salzsäure und 500 ml gesättigter wässriger Natriumchloridlösung gewaschen, über Magnesiumsulfat getrocknet und im Vakuum vom Lösungsmittel befreit.

Man erhält 142,8 g (93 % der Theorie) an 5-(N-Propionyl-N-methylamino)-3-methyl-4-trifluormethylsulfonyl-1-(2,6-dichlor-4-trifluormethylphenyl)-pyrazol als Öl, welches ohne zusätzliche Reinigung weiter umgesetzt wird.

Anwendungsbeispiele

In den folgenden Anwendungsbeispielen wurden die nachstehend aufgeführten Verbindungen als Vergleichsubstanzen eingesetzt:

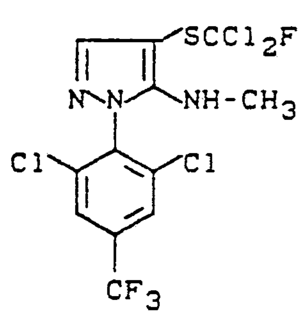

(A)

1-(2,6-Dichlor-4-trifluormethylphenyl)-5-methylamino-4-dichlorfluormethylthio-pyrazol

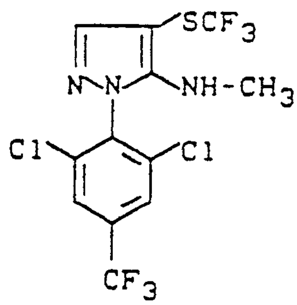

(B)

1-(2,6-Dichlor-4-trifluormethylphenyl)-5-methylamino-4-trifluormethylthio-pyrazol (beide bekannt aus EP 201 852)

Beispiel A

Mückenlarven-Test

Testtiere: 4. Larvenstadium Aedes aegypti
Lösungsmittel: 99 Gewichtsteile Aceton
Emulgator: 1 Gewichtsteil Benzylhydroxydiphenylpolyglykolether

Zur Herstellung einer zweckmäßigen Wirkstoffzubereitung löst man 2 Gew.-Teile Wirkstoff in 1000 Volumenteilen Lösungsmittel, das Emulgator in der oblen angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Kunststoffbecher und setzt anschließend 25 Mückenlarven in jeden Becher ein. Die Larven werden täglich mit Fischfutter (Tetramin®) gefüttert.

Nach 24 Stunden wird die Abtötung in % bestimmt. Dabei bedeutet 100 %, daß alle Larven abgetötet worden sind. 0 % bedeutet, daß überhaupt keine Larven abgetötet worden sind.

Bei diesem Test zeigen z.B. die folgenden Verbindungen der Herstellungsbeispiele überlegene Wirkung gegenüber dem Stand der Technik: 1.

Beispiel B

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1.

Beispiel C

Test mit Lucilia cuprina resistent-Larven

Emulgator:
35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber der Stand der Technik: 1.

Beispiel D

Test mit Boophilus microplus resistent /OP-resistenter Biarra-Stamm

Lösungsmittel:
35 Gewichtsteile Ethylenglykolmonomethylester
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1.

Beispiel E

Test mit parasitierenden, adulten Stechfliegen (Stomoxys calcitrans)

Lösungsmittel: Cremophor

Zur Herstellung einer zweckmäßigen Wirkstoffzubereitung vermischt man die betreffende aktive Substanz mit dem angegebenen Lösungsmittel im Verhältnis 1 : 2 und verdünnt das so erhaltene Konzentrat mit Wasser auf die gewünschte Konzentration.

10 adulte Stechfliegen (Stomoxys calcitrans) werden in Petrischalen, die Sandwiches enthalten, die einen Tag vor Versuchsbeginn mit 1 ml der zu testenden Wirkstoffzubereitung durchtränkt wurden, gebracht. Nach 3 Stunden wird der Abtötungsgrad bestimmt, wobei 100 % bedeuten, daß alle und 0 %, daß keine Fliege abgetötet worden ist.

Bei diesem Test zeigt z.B. die folgende Verbindung der Herstellungsbeispiele überlegene Wirkung gegenüber dem Stand der Technik: 1.

Beispiel F

Facefly - Test (Musca autumnalis)

Lösungsmittel:
35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Faceflies (Musca autumnalis) werden in Petrischalen gebracht, die Filterpapierscheiben entsprechender Größe enthalten, die einen Tag vor Versuchsbeginn mit 1 ml der zu testenden Wirkstffzubereitung durchtränkt wurden. Nach 3 Stunden wird der Abtötungsgrad in Prozent bestimmt, wobei 100% bedeuten, daß alle und 0%, daß keine Fliegen abgetötet worden sind.

Bei diesem Test zeigt z.B. die folgende Verbindung der Herstellungsbeispiele überlegene Wirkung gegenüber dem Stand der Technik: 1.

Beispiel G

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wird auf eine Replica-Platte gegeben. Dazu gibt man 2 ml einer E.coli-Suspension, zu der man 10 - 20 weibliche Tiere oder Larven von Caenorhabditis elegans in 0,5 ml steriler M9 Pufferlösung gegeben hat. Die E.coli-Suspension wird hergestellt, indem man 300 ml einer Übernachtkultur eines Uracil-bedürftigen E.coli-Stammes mit 1,8 l steriler M9 Pufferlösung versetzt.

Der Versuchsansatz wird 7 Tage bei 22 °C inkubiert und danach ausgewertet. Es wird bewertet, inwieweit der Wirkstoff die Vermehrung beeinträchtigt und die Konzentration angegeben bei der die Vermehrung verhindert wird.

Bei diesem Test zeigen z.B. die folgenden Verbindungen der Herstellungsbeispiele eine mindestens 95 %ige Hemmung der Vermehrung des Nematoden C. elegans
- bei einer Konzentration von ≦ 10µg/ml: 1

## Ansprüche

1. Substituierte 5-Methylamino-1-arylpyrazole der allgemeinen Formel (I)

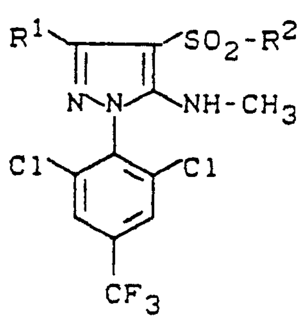

(I)

in welcher
$R^1$ für Alkyl steht und
$R^2$ für Halogenalkyl steht.

2. Substituierte 5-Methylamino-1-arylpyrazole der allgemeinen Formel (I) gemäß Anspruch 1, in welcher $R^1$ für geradkettiges oder verzweigtes Alkyl mit 1 bis 4 Kohlenstoffatomen steht und $R^2$ für geradkettiges oder verzweigtes Halogenalkyl mit 1 bis 4 Kohlenstoffatomen und 1 bis 9 gleichen oder verschiedenen Halogenatomen steht.

3. Substituierte 5-Methylamino-1-arylpyrazole der allgemeinen Formel (I) gemäß Anspruch 1, in welcher $R^1$ für Methyl oder Ethyl steht und $R^2$ für Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl , Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl oder Fluorchlorbrommethyl steht.

4. Substituierte 5-Methylamino-1-arylpyrazole der allgemeinen Formel (I) gemäß Anspruch 1, in welcher $R^1$ für Methyl steht und $R^2$ für Trifluormethyl, Dichlorfluormethyl oder Difluorchlormethyl steht.

5. Verfahren zur Herstellung von substituierten 5-Methylamino-1-arylpyrazolen der allgemeinen Formel (I)

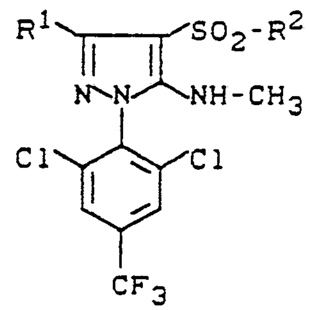

(I)

in welcher
$R^1$ für Alkyl steht und
$R^2$ für Halogenalkyl steht,
dadurch gekennzeichnet, daß man
(a) 5-Amino-1-aryl-pyrazole der Formel (II),

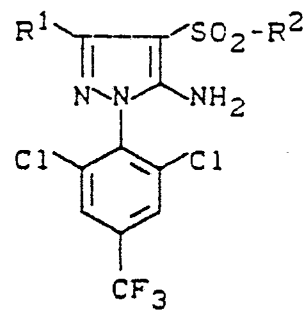

(II)

in welcher
$R^1$ und $R^2$ die oben angegebene Bedeutung haben,
mit Methylierungsmitteln der Formel (III),

$CH_3$-E (III)

in welcher
E für eine elektronenanziehende Abgangsgruppe steht,
gegebenenfalls in Gegenwart eines Verdünnungsmittels und gegebenenfalls in Gegenwart eines Säurebindemittels sowie gegebenenfalls in Gegenwart eines Katalysators umsetzt; oder daß man
(b) 5-(N-Acylamino)-1-arylpyrazole der Formel (IV),

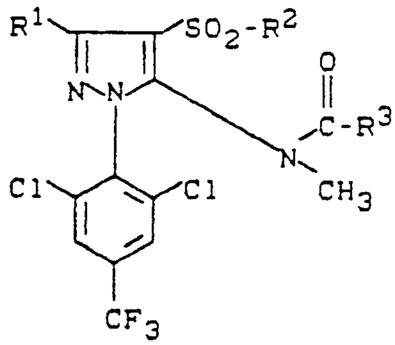

(IV)

in welcher
R' und $R^2$ die oben angegebene Bedeutung haben und
$R^3$ für Alkyl steht,
mit Säuren als Katalysator gegebenenfalls in Gegenwart eines Verdünnungsmittels deacyliert; oder daß man
(c) 1-Arylpyrazole der Formel (V),

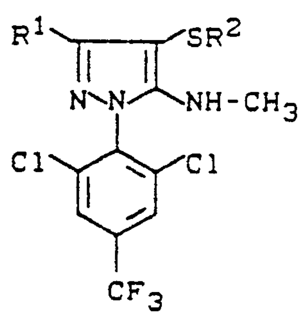

(V)

in welcher
R' und $R^2$ die oben angegebene Bedeutung haben,
mit einem Oxidationsmittel, gegebenenalls in Gegenwart eines Verdünnungsmittels und gegebenenfalls in Gegenwart eines Katalysators sowie gegebenenfalls in Gegenwart eines Säurebindemittels umsetzt; oder daß man
(d) 5-Halogen-1-aryl-pyrazole der Formel (VI),

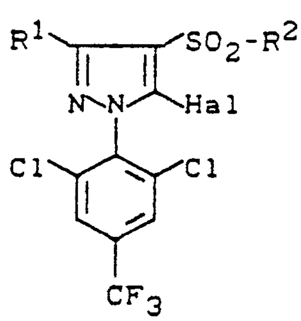

(VI)

in welcher
R' und $R^2$ die oben angegebene Bedeutung haben und
Hal für Halogen steht,
mit Methylamin der Formel (VII),

$CH_3$-$NH_2$ (VII)

gegebenenfalls in Gegenwart eines Verdünnungsmittel und gegebenenfalls in Gegenwart eines Säurebindemittels umsetzt.

6. Schädlingsbekämpfungsmittel, gekennzeichnet durch einen Gehalt an mindestens einem substituierten 5-Methylamino-1-arylpyrazol der Formel (I).

7. Verfahren zur Bekämpfung von tierischen Schädlingen, dadurch gekennzeichnet, daß man substituierte 5-Methylamino-1-arylpyrazole der Formel (I) auf tierische Schädlinge und/oder ihren Lebensraum einwirken läßt.

8. Verwendung von substituierten 5-Methyl-1-arylpyrazolen der Formel (I) zur Bekämpfung von tierischen Schädlingen.

9. Verfahren zur Herstellung von Schädlingsbekämpfungsmitteln, dadurch gekennzeichnet, daß man substituierte 5-Methylamino-1-arylpyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88 10 8603

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 201 852 (BAYER)<br>* Das ganze Dokument * | 1-4,6-9 | C 07 D 231/44<br>A 01 N 43/56 |
| P,X | EP-A-0 234 119 (MAY & BAKER)<br>* Das ganze Dokument * | 1-4,6-9 | |
| P,A | EP-A-0 260 521 (BAYER)<br>* Das ganze Dokument * | | |
| P,A | EP-A-0 235 628 (BAYER)<br>* Das ganze Dokument * | | |

RECHERCHIERTE SACHGEBIETE (Int. Cl.4)

C 07 D 231/00
A 01 N 43/00

Der vorliegende Recherchenbericht wurde für alle Patentansprüche erstellt

| Recherchenort | Abschlußdatum der Recherche | Prüfer |
|---|---|---|
| DEN HAAG | 18-09-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)